# EUROPEAN PATENT SPECIFICATION

(11) **EP 0 462 986 B1**
(45) Date of publication and mention of the grant of the patent: **06.10.1993**
(21) Application number: 90903371.4
(22) Date of filing: 27.02.1990
(51) Int. Cl.: C07D 487/04, C07D 513/04, A61K 31/425, A61K 31/505

(54) **IMIDAZOPYRIMIDINE ANTIALLERGY AGENTS**
IMIDAZOPYRIMIDINE, ANTIALLERGISCHE HEILMITTEL
AGENTS D'IMIDAZOPYRIMIDINE ANTIALLERGIE

(30) Priority: 07.03.1989 GB 8905130
(43) Date of publication of application: 02.01.1992
(73) Proprietor: Pfizer Limited, Sandwich Kent CT13 9NJ (GB); PFIZER INC., New York, N.Y. 10017 (US)
(72) Inventor: COOPER, Kelvin Southern View Cherry Lane, Deal Kent (GB)
(74) Representative: Moore, James William, Dr.
(86) International application number: EP9000322
(87) International publication number: WO9010632

(56) References cited:
- EP-A- 0 120 589
- EP-A- 0 197 230
- DE-A- 2 445 335
- FR-A- 2 510 576

## Description

### Technical Field

This invention relates to imidazopyrimidines, specifically to certain 4-aryl-5-alkoxycarbonyl-4,7-dihydro-imidazo[1,2-a]pyrimidines. The compounds are potent and selective antagonists of platelet activating factor having clinical utility in the treatment of allergic and inflammatory conditions in humans.

### Background Art

Platelet activating factor (PAF, 1-0-alkyl-2-acetyl-sn-glyceryl-3-phosphorylcholine) is an ether phospholipid whose structure was first elucidated in 1979. It is produced by, released from and interacts with many pro-inflammatory cells, platelets and the kidney. In addition to potent platelet aggregating activity, PAF exhibits a wide spectrum of biological activities elicited either directly or via the release of other powerful mediators such as thromboxane A₂ or the leukotrienes. In vitro, PAF stimulates the movement and aggregation of neutrophils and the release therefrom of tissue-damaging enzymes and oxygen radicals. These activities contribute to actions of PAF in vivo consistent with it playing a significant role in inflammatory and allergic responses. Thus, intradermal PAF has been shown to induce an inflammatory response, with associated pain, accumulation of inflammatory cells and increased vascular permeability, comparable with the allergic skin reaction following exposure to allergen. Similarly, both the acute broncho-constriction and chronic inflammatory reactions elicited by allergens in asthma can be mimicked by intratracheal administration of PAF. Accordingly agents which antagonise the actions of PAF and, consequently also prevent mediator release by PAF, will have clinical utility in the treatment of a variety of allergic, inflammatory and hypersecretory conditions such as asthma, arthritis, rhinitis, bronchitis and urticaria.

In addition to the above, PAF has been implicated as being involved in a number of other medical conditions. Thus in circulatory shock, which is characterised by systemic hypotension, pulmonary hypertension and increased lung vascular permeability, the symptoms can be mimicked by infusion of PAF. This, coupled with evidence showing that circulating PAF levels are increased by endotoxin infusion, indicates that PAF is a prime mediator in certain forms of shock. Intravenous infusion of PAF at doses of 20-200 pmol kg⁻¹ min⁻¹ into rats results in the formation of extensive haemorrhagic erosions in the gastric mucosa and thus PAF is the most potent gastric ulcerogen yet described whose endogenous release may underlie or contribute to certain forms of gastric ulceration. Psoriasis is an inflammatory and proliferative disease characterised by skin lesions. PAF is pro-inflammatory and has been isolated from lesioned scale of psoriatic patients indicating PAF has a role in the disease of psoriasis. Also increasing evidence supports a potential pathophysiological role for PAF in cardiovascular disease. Thus recent studies in angina patients show PAF is released during atrial pacing. Intracoronary injection of PAF in pigs induces a prolonged decrease in coronary flow and, in guinea pig hearts, it induces regional shunting and ischaemia. In addition PAF has been shown to initiate thrombus formation in a mesenteric artery preparation, both when administered exogenously and when released endogenously. More recently PAF has been shown to play a role in brain ischaemia induced in animal models of stroke.

Thus the compounds of the invention, by virtue of their ability to antagonise the actions of PAF, will be of value in the treatment of the above conditions.

Imidazopyrimidines have previously been disclosed for a variety of therapeutic uses. Thus EP-A-0120589 discloses certain imidazo-heterocyclic compounds, including
imidazo[1,2-a]pyrimidines as cardiotonic and antiulcer agents;
DE-A-2445335 discloses certain tetrahydro-imidazopyrimidines as analgesic agents;
FR-A-2510576 discloses certain derivatives of 2-phenylimidazo[1,2-a]pyrimidine as antiinflammatory agents;
EP-A-0197230 discloses certain 7-alkoxy or alkylthio-substituted imidazo[1,2-a]pyrimidines as anxiolytic agents, and US-A-4551530 discloses certain 4-pyridyl imidazo[1,2-a]pyrimidines as antihypertensive and anxiolytic agents.

### Disclosure of the Invention

According to the present invention there are provided compounds of the formula: 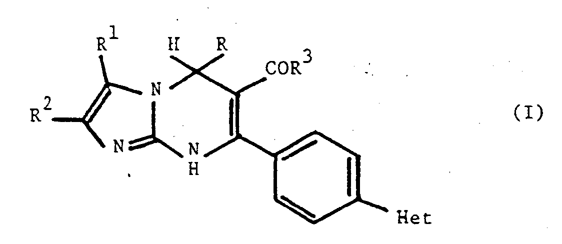
and their pharmaceutically acceptable salts,
- wherein: R is halophenyl;
R¹ and R² are each independently H or C₁-C₆ alkyl, or they are joined to complete a fused benzene ring;
R³ is C₁-C₄ alkoxy;
- and: Het is an imidazolyl, thiazolyl, oxazolyl, triazolyl, pyridyl, benzimidazolyl, imidazopyridyl or imidazothiazolyl group, any of these groups being optionally substituted in either ring by one or more substituents independently selected from C₁-C₄ alkyl, C₁-C₄ alkoxy, halo, trifluoromethyl and cyano.

In the definitions given herein, the term halo means fluoro, chloro, bromo or iodo. Alkyl and alkoxy groups of 3 or more carbon atoms may be straight or branched-chain.

Typical examples of Het are 2-methylimidazo[4,5-c]pyrid-1-yl, 2-trifluoromethylimidazo-[4,5-c]pyrid-1-yl, 2-n-butylimidazo[4,5-c]pyrid-1-yl, 3,5-dimethyl-1,2,4-triazol-4-yl, 2-methylimidazo[4,5-b]pyrid-1-yl, 2-methylimidazo[1,2-a]pyrid-3-yl, 2-methylbenzimidazol-1-yl, 2,4,5-trimethylimidazol-1-yl, 2,4-dimethylthiazol-5-yl, 2,4-dimethyloxazol-5-yl, 2,6-dimethylpyrid-3-yl, and 2-methylimidazo[1,2-b]thiazol-3-yl.

R is preferably 2-chlorophenyl; R³ is preferably ethoxy; R¹ and R² are preferably H and Het is most preferably 2-methylimidazo-[4,5-c]pyrid-1-yl.

A particularly preferred individual compound of the invention is 4-(2-chlorophenyl)-4,7-dihydro-5-ethoxycarbonyl-6-[4-(2-methylimidazo[4,5-c]pyrid-1-yl)phenyl]imidazo[1,2-a]pyrimidine.

The compounds of the formula (I) contain at least one asymmetric centre and exist as one or more pairs of enantiomers. Such pairs or individual isomers may be separable by physical methods, e.g. by fractional crystallisation or chromatography of the parent compounds or of a suitable salt or derivatives thereof. The invention includes all the enantiomers whether separated or not.

The pharmaceutically acceptable acid addition salts of the compounds of the formula (I) which form such salts are those formed from acids which form non-toxic acid addition salts, for example the hydrochloride, hydrobromide, sulphate or bisulphate, phosphate or acid phosphate, acetate, citrate, fumarate, gluconate, lactate, maleate, succinate and tartrate salts.

The compounds of formula I may be obtained according to the following reaction scheme: 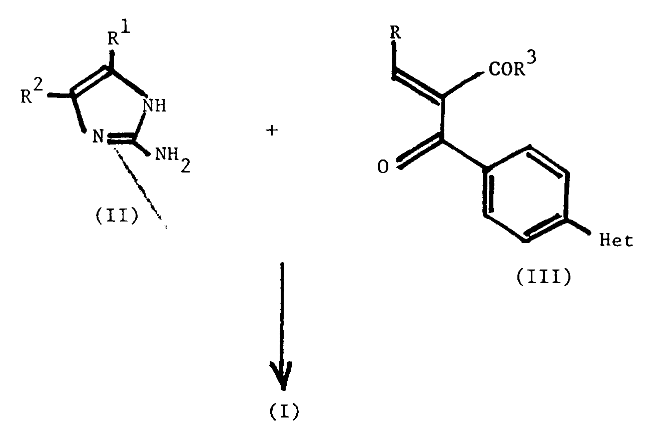
wherein R, R¹, R², R³, and Het are as previously defined.

In a typical procedure, the ketoester or ketoamide (III) and the amino-imidazole (II) are heated in a suitable organic solvent, e.g. ethanol or dimethylformamide, for several hours, optionally in the presence of a base, e.g. sodium bicarbonate. The product of formula (I) can then be isolated and purified by conventional procedures, for example by partition, recrystallisation or by chromatography.

Certain compounds of formula (I) are also conveniently obtained by means of simple chemical transformation reactions. Thus for example compounds of formula (I) wherein R³ is benzyloxy may be subjected to a conventional catalytic hydrogenation to yield the corresponding compounds wherein R³ is OH. The acid product may then be reacted with ammonia or with an amine in the presence of a diimide coupling agent, to yield the amide or substituted amide wherein R³ is NR⁴R⁵. Appropriate reagents and conditions for these transformations will be well known to those skilled in the art.

The ketoesters and ketoamides of formula (III) are either known compounds or can be prepared by methods analogous to those of the prior art, such as the method described in European patent 100189 which is essentially the method of Troostwijk and Kellogg, J.C.S. Chem. Comm., 1977, page 932, or as described in the Preparations given hereafter. Also the amino-imidazoles are either known or can be prepared by known methods in accordance with literature precedents.

The activity of the compounds of the invention is shown by their ability to inhibit the platelet aggregating activity of PAF in vitro. Testing is performed as follows:

Blood samples are taken from either rabbit or man into 0.1 vol disodium ethylenediamine tetraacetic acid buffer and the samples centrifuged for 15 minutes to obtain platelet rich plasma. The plasma is further centrifuged to give a platelet pellet which is washed with a buffer solution (4 mM KH₂PO₄, 6mM Na₂HPO₄, 100 mM NaCl, 0.1% glucose and 0.1% bovine serum albumin, pH 7.25) and finally resuspended in buffer solution to a concentration of 2 x 10⁸ platelets/ml. A sample (0.5 ml) is pre-incubated with stirring for two minutes at 37°C in a Paton aggregometer, either with vehicle alone, or with vehicle containing the particular compound under test. PAF is added at a sufficient concentration to give a maximum aggregating response in the absence of test compound (10⁻⁸ to 10⁻⁹ molar), and the platelet aggregation is measured by following the increase in light transmission of the solution. The experiment is repeated in the presence of test compound at a range of concentrations and the concentration of compound required to reduce the response to 50% of its maximum value is recorded as the IC₅₀ value.

The activity of the compounds of formula (I) is also demonstrated in vivo by their ability to protect mice from the lethal effect of an injection of PAF. A mixture of PAF (50 µg/kg) and DL-propranolol (5 mg/kg) in 0.9% w/v sodium chloride is injected (0.2 ml) via a tail vein into mice. The compounds under test are either injected into the tail vein immediately prior to the PAF/propranolol injection or administered orally by gavage two hours earlier. The compounds are tested at several doses in groups of 5 mice and the dose which reduces mortality to 50% is recorded as the PD₅₀ value.

The compounds are also tested for their ability to reduce PAF-induced bronchoconstriction in anaesthetised guinea pigs. In this test airways resistance and dynamic lung compliance are calculated from recordings of airflow and transpleural pressure and calculation of tidal volume. The bronchoconstriction induced by PAF (100 ng/kg) is determined. One hour after the initial dose of PAF the compound under test is administered and the PAF challenge repeated. The ability of the compound to reduce the bronchoconstrictor effect of PAF is calculated as a ratio.

For therapeutic use the compounds of the formula (I) will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For administration to man in the curative or prophylactic treatment of allergic bronchial conditions and arthritis, oral dosages of the compounds will generally be in the range of from 2-1000 mg daily for an average adult patient (70 kg). Thus for a typical adult patient, individual tablets or capsules contain from 1 to 500 mg of active compound, in a suitable pharmaceutically acceptable vehicle or carrier. Dosages for intravenous administration would typically be within the range 1 to 10 mg per single dose as required. For the treatment of allergic and bronchial hyper-reactive conditions, inhalation via a nebuliser or aerosol may be the preferred route of drug administration. Dose levels by this route would be within the range 0.1 to 50 mg per single dose as required. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

Thus in a further aspect the invention provides a pharmaceutical composition comprising a compound of the formula (I), or a pharmaceutically acceptable salt thereof, together with a pharmaceutically acceptable diluent or carrier.

The invention also includes a compound of the formula (I), or a pharmaceutically acceptable salt thereof, for use in medicine, in particular in the treatment of allergic, inflammatory and hypersecretory conditions in a human being.

The preparation of the compounds of the invention is further illustrated by the following Examples.

### EXAMPLE 1

4-(2-Chlorophenyl)-4,7-dihydro-5-ethoxycarbonyl-6-[4-(2-methylimidazo[4,5-c]pyrid-1-yl)phenyl]imidazo[1,2-a]pyrimidine

A mixture of 2-aminoimidazole bisulphate (120 mg), 3-(2-chlorophenyl)-2-ethoxycarbonyl-1-[4-(2-methylimidazo[4,5-c]pyrid-1-yl)phenyl]prop-2-ene-1-one(200 mg) and sodium bicarbonate (240 mg) was heated at 70°C in dimethylformamide (5 ml) for 4 hours. The cooled mixture was poured into water (50 ml) and extracted with ethyl acetate (3 x 30 ml). The combined organic phases were dried (Na₂SO₄), filtered and evaporated and the residue chromatographed on silica eluting with ethyl acetate/methanol. The fractions containing product were combined and evaporated to give the title compound (70 mg), m.p. 205-210°C. Found: C,65.34; H,4.94; N,16.3. C₂₈H₂₃ClN₆O₂0.25 H₂O requires C,65.24; H,4.59; N,16.3%.

### EXAMPLE 2

4-(2-Chlorophenyl)-4,7-dihydro-2,3-dimethyl-5-ethoxycarbonyl-6-[4-(2-methylimidazo[4,5-c]pyrid-1-yl)-phenyl]imidazo[1,2-a]-pyrimidine

The title compound was prepared following the procedure of Example 1 using 2-amino-4,5-dimethylimidazole as starting material. The product was obtained as a white solid m.p. 191-197°C. Found: C,65.75; H,5.15; N,15.33. C₃₀H₂₇ClN₆O₂. 0.5H₂0 requires C,65.59; H,5.17; N,15.11%.

### EXAMPLE 3

4-(2-Chlorophenyl)-4,7-dihydro-5-ethoxycarbonyl-6-[4-(2-methyl-imidazo[4,5-c]pyrid-1-yl)phenyl]benzoimidazo[1,2-a]pyrimidine

A mixture of 2-aminobenzimidazole (70 mg) and 3-(2-chlorophenyl)-2-ethoxycarbonyl-1-[4-(2-methylimidazo[4,5-c]pyrid-1-yl)phenyl]prop-2-ene-1-one (220 mg) was heated at reflux in ethanol (10 ml) for 2 hours. After cooling the precipitated solid was collected by filtration and then chromatographed on silica eluting with ethyl acetate/methanol to give the title compound (90 mg), m.p. 210-217°C. Found: C,65.36; H,4.82; N,14.12. C₃₂H₂₅ClN₆0₂. 1.5 H₂0 requires C,65.36; H,4.8; N,14.29%.

### PREPARATION

3-(2-Chlorophenyl)-2-ethoxycarbonyl-1-[4-(2-methylimidazo[4,5-c]pyrid-1-yl)phenyl]-prop-2-ene-1-one.
(a) 1-(4-Cyanophenyl)-2-methylimidazo[4,5-c]pyridine
   A mixture of 3-amino-4-(4-cyanophenyl)aminopyridine (9.31 g, 44.3 mmol), triethyl-orthoacetate (40 ml) and acetic anhydride (30 ml) was heated at reflux for 2 hours under nitrogen, cooled, then concentrated under reduced pressure. The brown residue was dissolved in 1M hydrochloric acid and washed with ethyl acetate (200 ml). The aqueous layer was rendered basic with saturated aqueous ammonia and extracted with dichloromethane (3 x 200 ml). The combined extracts were washed with water, dried (MgSO₄) and concentrated to give 1-(4-cyanophenyl)-2-methylimidazo[4,5-c]pyridine, 6.5 g as a brown solid.
(b) Ethyl 3-[4-(2-methylimidazo[4,5-c]pyrid-1-yl)phenyl]-3-ketopropanoate
   Zinc dust (894 mg, 13.7 mmol) was suspended in dry tetrahydrofuran (3 ml) under nitrogen and sonicated at room temperature for 10 minutes. Ethyl bromoacetate (2 drops) was added and the mixture was refluxed for 5 minutes. A solution of 1-(4-cyanophenyl)-2-methylimidazo[4,5-c]pyridine (640 mg, 2.74 mmol) in dry tetrahydrofuran (6 ml) was added and the mixture was refluxed for 5 minutes. A solution of ethyl bromoacetate (1.822 g, 10.94 mmol) in dry tetrahydrofuran (2 ml) was added dropwise over 1 hour at reflux, and after a further 10 minutes, the mixture was allowed to cool to room temperature. 50% Aqueous potassium carbonate (1 ml) was added and the mixture was stirred for 45 minutes at room temperature, and then filtered washing with tetrahydrofuran. The filtrate was concentrated under reduced pressure to give a yellow gum. This material was treated with a mixture of 20% aqueous trifluoroacetic acid (10 ml) and dichloromethane (50 ml) at room temperature for 15 minutes. The mixture was neutralised by the addition of saturated aqueous sodium hydrogen carbonate, and then extracted with dichloromethane (2 x 30 ml). The combined extracts were dried (MgSO₄), concentrated under reduced pressure, and the crude product was purified by flash chromatography (eluting with 10 - 20% methanol in ethyl acetate to give the title product (480 mg, 54%) as a yellow gum.
(c) 3-(2-Chlorophenyl)-2-ethoxycarbonyl-1-[4-(2-methylimidazo[4,5-c]-pyrid-1-yl)phenyl]-prop-2-ene-1-one.
   A mixture of 2-chlorobenzaldehyde (2.8 g) ethyl-3-[4-(2-methylimidazo[4,5-c]pyrid-1-yl)phenyl]-3-ketopropanoate (6.4 g) and piperidine (100µl) were stirred at room temperature for 48 hours in acetonitrile (30 ml). The mixture was evaporated to dryness and the residue chromatographed on silica eluting with ethyl acetate/methanol (5:1). The fractions containing product were combined and evaporated to give the title compound (5.3 g). N.M.R. (δ) 1.35(t,3H); 2.53(s,3H); 4.27(q,2H), 7.0-9.1 (m,12H).

## Claims (Claims for the following Contracting State(s): AT, BE, CH, DE, DK, FR, GB, IT, LI, LU, NL, SE)

1. A compound having the formula: 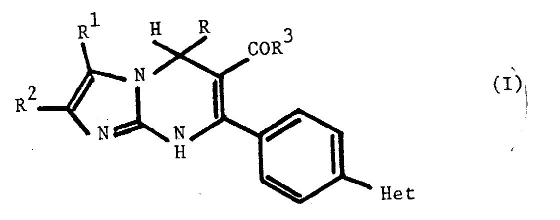 and pharmaceutically acceptable salts thereof
wherein R is halophenyl;
R¹ and R² are each independently H or C₁-C₆ alkyl, or they are joined to complete a fused benzene ring;
R³ is C₁-C₄ alkoxy;
and Het is an imidazolyl, thiazolyl, oxazolyl, triazolyl, pyridyl, benzimidazolyl, imidazopyridyl or imidazothiazolyl group, any of these groups being optionally substituted in either ring by one or more substituents independently selected from C₁-C₄ alkyl, C₁-C₄ alkoxy, halo, trifluoromethyl and cyano.

2. A compound as claimed in claim 1 wherein R is 2-chlorophenyl.

3. A compound as claimed in claim 1 wherein R³ is ethoxy.

4. A compound as claimed in claim 1 wherein Het is 2-methylimidazo[4,5-c]pyrid-1-yl, 2-trifluoromethylimidazo[4,5-c]pyrid-1-yl, 2-n-butylimidazo[4,5-c]pyrid-1-yl, 3,5-dimethyl-1,2,4-triazol-4-yl, 2-methylimidazo[4,5-b]pyrid-1-yl, 2-methylimidazo[1,2-a]-pyrid-3-yl, 2-methylbenzimidazol-1-yl, 2,4,5-trimethylimidazol-1-yl, 2,4-dimethylthiazol-5-yl, 2,4-dimethyloxazol-5-yl, 2,6-dimethylpyrid-3-yl, or 2-methylimidazo[1,2-b]thiazol-3-yl.

5. A compound as claimed in claim 1 wherein Het is 2-methylimidazo[4,5-c]pyrid-1-yl.

6. A compound as claimed in claim 1 wherein R is 2-chlorophenyl, R³ is ethoxy, Het is 2-methylimidazo[4,5-c]pyrid-1-yl and R¹ and R² are either both H, or they are both CH₃ or they are joined to complete a fused benzene ring.

7. A compound as claimed in claim 1 wherein said compound is 4-(2-chlorophenyl)-4,7-dihydro-5-ethoxycarbonyl-6-[4-(2-methylimidazo[4,5-c]pyrid-1-yl)phenyl]imidazo[1,2-a]pyrimidine.

8. A process for preparing a compound of the formula (I) as defined in claim 1 which comprises reacting compounds of the formulae: 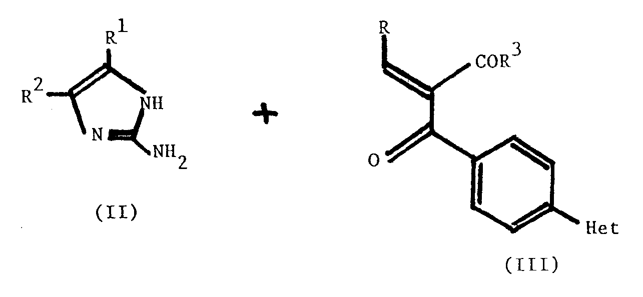 wherein R, R¹, R², R³ and Het are as previously defined in claim 1, by heating in an organic solvent, and isolating the compound of formula I.

9. A pharmaceutical composition comprising a compound of the formula (I) or a pharmaceutically acceptable salt thereof as claimed in any one of claims 1 to 7, together with a pharmaceutically acceptable diluent or carrier.

10. A compound of the formula (I) or a pharmaceutically acceptable salt thereof as claimed in any one of claims 1 to 7 for use in medicine, in particular for use in the treatment of allergic, inflammatory and hypersecretory conditions in humans.

## Claims (Claims for the following Contracting State(s): ES)

1. A process for preparing a compound having the formula (I) and pharmaceutically acceptable salts thereof, 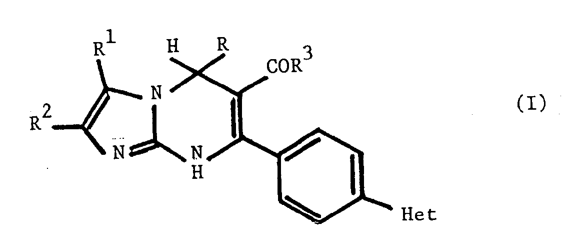
wherein R is halophenyl;
R¹ and R² are each independently H or C₁-C₆ alkyl, or they are joined to complete a fused benzene ring;
R³ is C₁-C₄ alkoxy;
and Het is an imidazolyl, thiazolyl, oxazolyl, triazolyl, pyridyl, benzimidazolyl, imidazopyridyl or imidazothiazolyl group, any of these groups being optionally substituted in either ring with one or more substituents independently selected from C₁-C₄ alkyl, C₁-C₄ alkoxy, halo, trifluoromethyl and cyano; which comprises reacting compounds of the formulae: 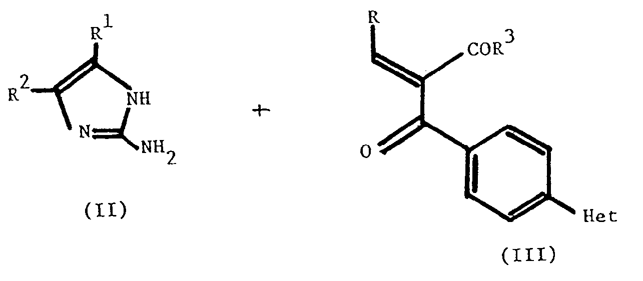
wherein R, R¹, R², R³ and Het are as previously defined, by heating in an organic solvent and isolating the compound of formula I.

2. A process as claimed in claim 1 wherein R is 2-chlorophenyl.

3. A process as claimed in claim 1 or 12 wherein R³ is ethoxy.

4. A process as claimed in any of claims 1 to 3 wherein Het is 2-methylimidazo[4,5-c]pyrid-1-yl, 2-n-trifluoromethylimidazo[4,5-c]-pyrid-1-yl, 2-n-butylimidazo[4,5-c]pyrid-1-yl, 3,5-dimethyl-1,2,4-triazol-4-yl, 2-methylimidazo[4,5-b]pyrid-1-yl, 2-methylimidazo[1,2-a]-pyrid-3-yl, 2-methylbenzimidazol-1-yl, 2,4,5-trimethylimidazol-1-yl, 2,4-dimethylthiazol-5-yl, 2,4-dimethyloxazol-5-yl, 2,6-dimethylpyrid-3-yl, or 2-methylimidazo[1,2-b]thiazol-3-yl.

5. A process as claimed in claim 4 wherein Het is 2-methylimidazo[4,5-c]pyrid-1-yl.

6. A process as claimed in claim 1 wherein R is 2-chlorophenyl, R³ is ethoxy, Het is 2-methylimidazo[4,5-c]pyrid-1-yl and R¹ and R² are either both H, or they are both CH₃ or they are joined to complete a fused benzene ring.

7. A process as claimed in claim 1 wherein said compound of formula (I) is 4-(2-chlorophenyl)-4,7-dihydro-5-ethoxycarbonyl-6-[4-(2-methyl-imidazo[4,5-c]pyrid-1-yl)phenyl]imidazo[1,2-a]pyrimidine.

8. A process for preparing a pharmaceutical composition characterised by mixing a compound of the formula (I) as defined in claim 1, or a pharmaceutically acceptable salt thereof, with a pharmaceutically acceptable diluent or carrier.

## Patentansprüche (Patentansprüche für folgende(n) Vertragsstaat(en): AT, BE, CH, DE, DK, FR, GB, IT, LI, LU, NL, SE)

1. Verbindung der Formel: 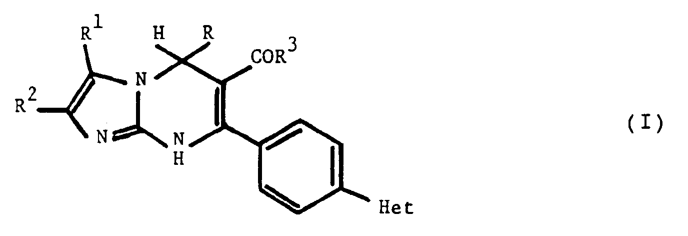 und pharmazeutisch verträgliche Salze davon,
worin R halogeniertes Phenyl bedeutet;
R¹ und R² jeweils unabhängig voneinander H oder C₁-C₆-Alkyl bedeuten oder zusammengenommen einen kondensierten Benzolring vervollständigen;
R³ C₁-C₄-Alkoxy bedeutet;
und Het eine Imidazolyl-, Thiazolyl-, Oxazolyl-, Triazolyl-, Pyridyl-, Benzimidazolyl-, Imidazopyridyl- oder Imidazothiazolylgruppe bedeutet, wobei
jede dieser Gruppen gegebenenfalls am Ring mit einem oder mehreren Substituenten, unabhängig voneinander ausgewählt aus C₁-C₄-Alkyl, C₁-C₄-Alkoxy, Halogen, Trifluormethyl und Cyano, substituiert ist.

2. Verbindung nach Anspruch 1, wobei R 2-Chlorphenyl bedeutet.

3. Verbindung nach Anspruch 1, wobei R³ Ethoxy bedeutet.

4. Verbindung nach Anspruch 1, wobei Het bedeutet: 2-Methylimidazo[4,5-c]pyrid-1-yl, 2-Trifluormethylimidazo[4,5-c]pyrid-1-yl, 2-n-Butylimidazo[4,5-c]pyrid-1-yl, 3,5-Dimethyl-1,2,4-triazol-4-yl, 2-Methylimidazo[4,5-b]pyrid-1-yl, 2-Methylimidazo[1,2-a]pyrid-3-yl, 2-Methylbenzimidazol-1-yl, 2,4,5-Trimethylimidazol-1-yl, 2,4-Dimethylthiazol-5-yl, 2,4-Dimethyloxazol-5-yl, 2,6-Dimethylpyrid-3-yl oder 2-Methylimidazo[1,2-b]thiazol-3-yl.

5. Verbindung nach Anspruch 1, wobei Het 2-Methylimidazo[4,5-c]pyrid-1-yl bedeutet.

6. Verbindung nach Anspruch 1, wobei R 2-Chlorphenyl bedeutet, R³ Ethoxy darstellt, Het 2-Methylimidazo[4,5-c]pyrid-1-yl bedeutet und R¹ und R² entweder beide H bedeuten oder beide CH₃ sind oder zusammengenommen einen kondensierten Benzolring vervollständigen.

7. Verbindung nach Anspruch 1 nämlich 4-(2-Chlorphenyl)-4,7-dihydro-5-ethoxycarbonyl-6-[4-(2-methylimid-azo[4,5-c]pyrid-1-yl)phenyl]imidazo[1,2-a]pyrimidin.

8. Verfahren zur Herstellung einer Verbindung der Formel (I) gemäß Anspruch 1, umfassend die Umsetzung der Verbindungen der Formeln: 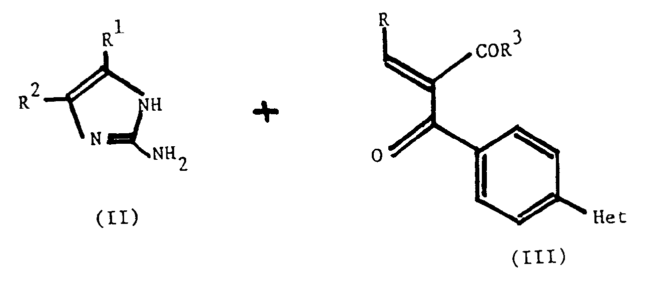 wobei R, R¹, R², R³ und Het wie vorstehend in Anspruch 1 definiert sind, durch Erhitzen in einem organischen Lösungsmittel und Gewinnen der Verbindung von Formel I.

9. Arzneimittel, umfassend eine Verbindung der Formel (I) oder ein pharmazeutisch verträgliches Salz davon, gemäß einem der Ansprüche 1 bis 7, zusammen mit einem pharmazeutisch verträglichen Verdünnungs- oder Trägermittel.

10. Verbindung der Formel (I) oder ein pharmazeutisch verträgliches Salz davon, gemäß einem der Ansprüche 1 bis 7, zur Verwendung als Arzneimittel, insbesondere bei der Behandlung von allergischen, entzündlichen und hypersekretorischen Zuständen beim Menschen.

## Patentansprüche (Patentansprüche für folgende(n) Vertragsstaat(en): ES)

1. Verfahren zur Herstellung einer Verbindung der Formel (I) 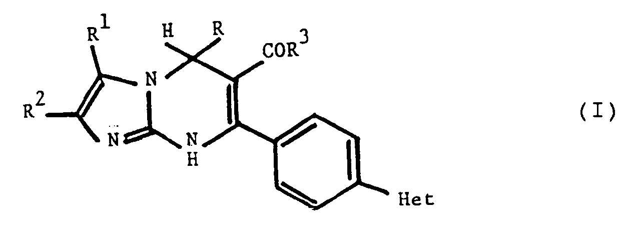 und eines pharmazeutisch verträglichen Salzes davon
worin
R halogeniertes Phenyl bedeutet;
R¹ und R² jeweils unabhängig voneinander H oder C₁-C₆-Alkyl bedeuten oder zusammengenommen einen kondensierten Benzolring vervollständigen;
R³ C₁-C₄-Alkoxy bedeutet;
und
Het eine Imidazolyl-, Thiazolyl-, Oxazolyl-, Triazolyl-, Pyridyl-, Benzimidazolyl-, Imidazopyridyl- oder Imidazothiazolylgruppe bedeutet, wobei
jede dieser Gruppen gegebenenfalls am Ring mit einem oder mehreren Substituenten, unabhängig voneinander ausgewählt aus C₁-C₄-Alkyl, C₁-C₄-Alkoxy, Halogen, Trifluormethyl und Cyano, substituiert ist; umfassend die Umsetzungen der Verbindungen der Formeln: 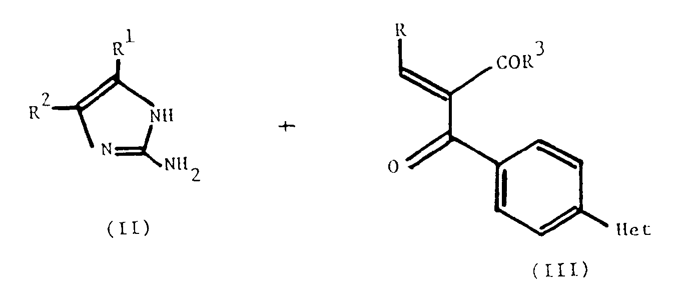 wobei R, R¹, R², R³ und Het wie vorstehend definiert sind, durch Erhitzen in einem organischen Lösungsmittel und Gewinnen der Verbindung von Formel I.

2. Verfahren nach Anspruch 1, wobei R 2-Chlorphenyl bedeutet.

3. Verfahren nach Anspruch 1 oder 2, wobei R³ Ethoxy bedeutet.

4. Verfahren nach Anspruch 1 bis 3, wobei Het bedeutet: 2-Methylimidazo[4,5-c]pyrid-1-yl, 2-n-Trifluormethylimidazo[4,5-c]pyrid-1-yl, 2-n-Butylimidazo[4,5-c]pyrid-1-yl, 3,5-Dimethyl-1,2,4-triazol-4-yl, 2-Methylimidazo[4,5-b]pyrid-1-yl, 2-Methylimidazo[1,2-a]pyrid-3-yl, 2-Methylbenzimidazol-1-yl, 2,4,5-Trimethylimidazol-1-yl, 2,4-Dimethylthiazol-5-yl, 2,4-Dimethyloxazol-5-yl, 2,6-Dimethylpyrid-3-yl oder 2-Methylimidazo[1,2-b]thiazol-3-yl.

5. Verfahren nach Anspruch 4, wobei Het 2-Methylimidazo[4,5-c]pyrid-1-yl bedeutet.

6. Verfahren nach Anspruch 1, wobei R 2-Chlorphenyl bedeutet, R³ Ethoxy darstellt, Het 2-Methylimidazo[4,5-c]pyrid-1-yl bedeutet und R¹ und R² entweder beide H bedeuten oder beide CH₃ sind oder zusammengenommen einen kondensierten Benzolring vervollständigen.

7. Verfahren nach Anspruch 1, wobei die Verbindung der Formel (I) 4-(2-Chlorphenyl)-4,7-dihydro-5-ethoxycarbonyl-6-[4-(2-methylimidazo[4,5-c]pyrid-1-yl)phenyl]imidazo[1,2-a]pyrimidin ist.

8. Verfahren zur Herstellung eines Arzneimittels, umfassend das Vermischen einer Verbindung der Formel (I) gemäß Anspruch 1, oder eines pharmazeutisch verträglichen Salzes davon mit einem pharmazeutisch verträglichen Verdünnungs- oder Trägermittel.

## Revendications (Revendications pour l'(les) Etat(s) contractant(s) suivant(s): AT, BE, CH, DE, DK, FR, GB, IT, LI, LU, NL, SE)

1. Composé de formule : 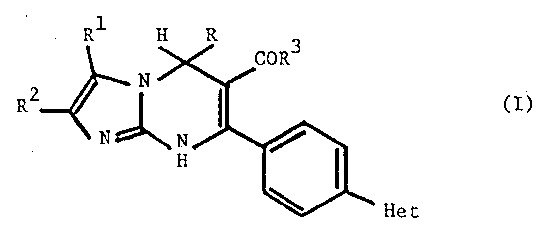 et sels pharmaceutiquement acceptables d'un tel composé, dans lesquels :
R est un groupe halogénophényle .
R¹ et R² représentent chacun, indépendamment l'un de l'autre, H ou un groupe alkyle en C₁-C₆, ou ils sont réunis pour compléter un noyau benzénique fusionné .
R³ est un groupe alcoxy en C₁-C₄ ;
et Het est un groupe imidazolyle, thiazolyle, oxazolyle, triazolyle, pyridyle, benzimidazolyle, imidazopyridyle ou imidazothiazolyle, un quelconque, ou plusieurs, de ces groupes étant éventuellement substitué sur l'un ou l'autre cycle par un ou plusieurs substituants sélectionnés, indépendamment l'un de l'autre, parmi les groupes alkyle en C₁-C₄, alcoxy en C₁-C₄, halogéno, trifluorométhyle et cyano.

2. Composé selon la revendication 1, dans lequel R est un groupe 2-chlorophényle.

3. Composé selon la revendication 1, dans lequel R³ est un groupe éthoxy.

4. Composé selon la revendication 1, dans lequel Het est un groupe 2-méthylimidazo(4,5-c)pyrid-1-yle, 2-trifluorométhylimidazo(4,5-c)pyrid-1-yle, 2-n-butylimidazo(4,5-c)pyrid-1-yle, 3,5-diméthyl-1,2,4-triazol-4-yle, 2-méthylimidazo(4,5-b)pyrid-1-yle, 2-méthylimidazo(1,2-a)pyrid-3-yle, 2-méthylbenzimidazol-1-yle, 2,4,5-triméthylimidazol-1-yle, 2,4-diméthylthiazol-5-yle, 2,4-diméthyloxazol-5-yle, 2,6-diméthylpyrid-3-yle, ou 2-méthylimidazo(1,2-b)thiazol-3-yle.

5. Composé selon la revendication 1, dans lequel Het est un groupe 2-méthylimidazo(4,5-c)pyrid-1-yle.

6. Composé selon la revendication 1, dans lequel R est un groupe 2-chlorophényle, R³ est un groupe éthoxy, Het est un groupe 2-méthylimidazo(4,5-c)pyrid-1-yle et R¹ et R² sont soit tous les deux H, soit tous les deux CH₃, soit réunis pour compléter un noyau benzénique fusionné.

7. Composé selon la revendication 1, dans lequel ledit composé est la 4-(2-chlorophényl)-4,7-dihydro-5-éthoxycarbonyl-6-(4-(2-méthylimidazo(4,5-c)pyrid-1-yl)phényl)imidazo(1,2-a)pyrimidine.

8. Procédé de préparation d'un composé de formule (I) tel que défini dans la revendication 1, qui consiste à faire réagir des composés de formules : 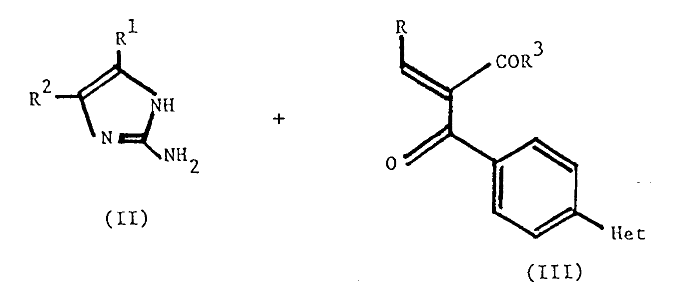 dans lesquelles R, R¹, R², R³ et Het sont tels que précédemment définis dans la revendication 1, par chauffage dans un solvant organique, et à isoler le composé de formule (I).

9. Composition pharmaceutique comprenant un composé de formule (I) ou un sel pharmaceutiquement acceptable d'un tel composé selon l'une quelconque des revendications 1 à 7, avec un diluant ou un véhicule pharmaceutiquement acceptable.

10. Composé de formule (I) ou sel pharmaceutiquement acceptable d'un tel composé selon l'une quelconque des revendications 1 à 7, destiné à être utilisé en médecine, en particulier à être utilisé dans le traitement des états allergiques inflammatoires et hypersécrétoires chez l'homme.

## Revendications (Revendications pour l'(les) Etat(s) contractant(s) suivant(s): ES)

1. Procédé de préparation d'un composé de formule (I) et de sels pharmaceutiquement acceptables d'un tel composé : 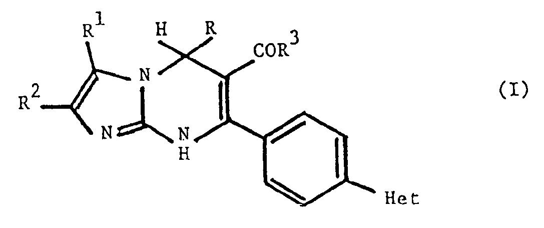 dans laquelle :
R est un groupe halogénophényle .
R¹ et R² représentent chacun, indépendamment l'un de l'autre, H ou un groupe alkyle en C₁-C₆, ou ils sont réunis pour compléter un noyau benzénique fusionné .
R³ est un groupe alcoxy en C₁-C₄ ;
et Het est un groupe imidazolyle, thiazolyle, oxazolyle, triazolyle, pyridyle, benzimidazolyle, imidazopyridyle ou imidazothiazolyle, un quelconque, ou plusieurs, de ces groupes étant éventuellement substitué sur l'un ou l'autre cycle par un ou plusieurs substituants sélectionnés, indépendamment l'un de l'autre, parmi les groupes alkyle en C₁-C₄, alcoxy en C₁-C₄, halogéno, trifluorométhyle et cyano, qui consiste à faire réagir des composés de formules : 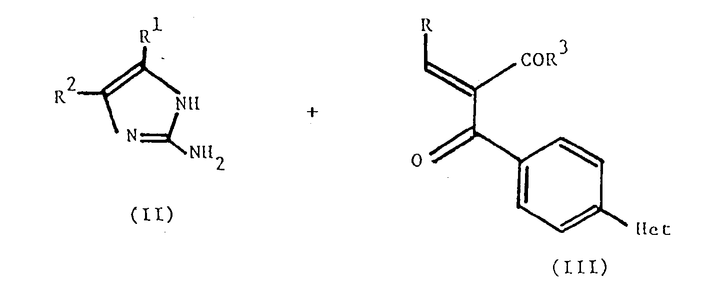 dans lesquelles R, R¹, R², R³ et Het sont tels que précédemment définis, par chauffage dans un solvant organique, et à isoler le composé de formule (I).

2. Procédé selon la revendication 1, dans lequel R est un groupe 2-chlorophényle.

3. Procédé selon la revendication 1 ou 2, dans lequel R³ est un groupe éthoxy.

4. Procédé selon l'une quelconque des revendications 1 à 3, dans lequel Het est un groupe 2-méthylimidazo(4,5-c)pyrid-1-yle, 2-trifluorométhylimidazo(4,5-c)pyrid-1-yle, 2-n-butylimidazo(4,5-c)pyrid-1-yle, 3,5-diméthyl-1,2,4-triazol-4-yle, 2-méthylimidazo(4,5-b)pyrid-1-yle, 2-méthylimidazo(1,2-a)pyrid-3-yle, 2-méthylbenzimidazol-1-yle, 2,4,5-triméthylimidazol-1-yle, 2,4-diméthylthiazol-5-yle, 2,4-diméthyloxazol-5-yle, 2,6-diméthylpyrid-3-yle, ou 2-méthylimidazo(1,2-b)thiazol-3-yle.

5. Procédé selon la revendication 4, dans lequel Het est un groupe 2-méthylimidazo(4,5-c)pyrid-1-yle.

6. Procédé selon la revendication 1, dans lequel R est un groupe 2-chlorophényle, R³ est un groupe éthoxy, Het est un groupe 2-méthylimidazo(4,5-c)pyrid-1-yle et R¹ et R² sont soit tous les deux H, soit tous les deux CH₃, soit réunis pour compléter un noyau benzénique fusionné.

7. Procédé selon la revendication 1, dans lequel ledit composé est la 4-(2-chlorophényl)-4,7-dihydro-5-éthoxycarbonyl-6-(4-(2-méthylimidazo(4,5-c)pyrid-1-yl)phényl)imidazo(1,2-a)pyrimidine.

8. Procédé de préparation d'une composition pharmaceutique caractérisée par le mélange d'un composé de formule (I) tel que défini dans la revendication 1, ou d'un sel pharmaceutiquement acceptable d'un tel composé, avec un diluant ou un véhicule pharmaceutiquement acceptable.
